# EUROPÄISCHE PATENTANMELDUNG

(11) **EP 1 733 695 A1**
(43) Veröffentlichungstag der Anmeldung: **20.12.2006**
(21) Anmeldenummer: 05012988.1
(22) Anmeldetag: 16.06.2005
(51) Int. Cl.: A61C 1/08, A61B 17/17

(54) **Abstandhalter für eine implantologische Führungseinrichtung und implantologische Führungseinrichtung**

(71) Anmelder: W & H Dentalwerk Bürmoos GmbH, 5111 Bürmoos (AT)
(72) Erfinder: Lette, Andreas, 4050 Traun (AT); Brandner, Jürgen, 5113 St. Georgen (AT)

(57) **Zusammenfassung**

Die vorliegende Erfindung betrifft einen Abstandhalter (1, 1') zur Bestimmung der Position einer Implantatkavität (54A, 54B), in die ein Dentalimplantat (70 - 72) einsetzbar ist, und eine implantologische, insbesondere kieferchirurgische Führungseinrichtung (25) mit einem Abstandhalter (1, 1') zur Führung eines in einem Dentalinstrument (51) aufgenommenen Bohrwerkzeugs (50) zum Bohren der Implantatkavität (54A, 54B). Erfindungsgemäß ist der feststehende Abstand zwischen der Positioniervorrichtung (2, 2') und der Stützvorrichtung (5, 5') für das Bohrwerkzeug (50) des Abstandhalters (1, 1') eine Funktion der Abmessung, d.h. des oder der Durchmesser (L₁, L₂, L₃), der zu setzenden Implantate (70 - 72), und der biologischen Breite (bB) zwischen den Zähnen und / oder Zahnersätzen, wodurch ein zuverlässigerer und natürlicherer Sitz des Zahnersatzes erreicht wird. Die Führungseinrichtung (25) ist mit dem Abstandhalter (1, 1') verbunden und gewährleistet durch eine Führungshülse (31), Führungsstifte (35), eine Schiebebuchse (38) und eine Aufnahmevorrichtung (40) für das Dentalinstrument (51) eine einfache und sichere Durchführung des Kavitätenbohrens für den Anwender.

Weiters betrifft die vorliegende Erfindung ein Werkzeugset (62) für Werkzeuge zum Bohren von Implantatkavitäten (54A, 54B) mit einem erfindungsgemäßen Abstandhalter (1, 1'), ein Hilfsmittel (57) zur Auswahl eines erfindungsgemäßen Abstandhalters (1, 1') und ein Verfahren zur Herstellung eines derartigen Abstandhalters (1, 1').

## Beschreibung

Die vorliegende Erfindung betrifft einen Abstandhalter zur Bestimmung der Position einer Implantatkavität, in die ein Dentalimplantat einsetzbar ist, nach dem Oberbegriff des Anspruchs 1 und eine implantologische Führungseinrichtung mit einem derartigen Abstandhalter zur Führung eines in einem Dentalinstrument aufgenommenen Bohrwerkzeugs zum Bohren der Implantatkavität. Weiters betrifft die vorliegende Erfindung ein Werkzeugset für Werkzeuge zum Bohren von Implantatkavitäten mit einem derartigen Abstandhalter, ein Hilfsmittel zur Auswahl eines erfindungsgemäßen Abstandhalters und ein Verfahren zur Herstellung eines derartigen Abstandhalters.

Ein derartiger Abstandhalter ist aus der EP 689 804 A1 bekannt. Er hat die Aufgabe beim Bohren von zwei oder mehr benachbarten Bohrungen (Implantatkavitäten) in ein Kiefer, in die anschließend Implantate als Basis für einen dentalen Zahnersatz (Krone) eingeschraubt werden, den Abstand zwischen den benachbarten Implantatkavitäten festzulegen und während des Bohrens anzuzeigen. Der Abstandhalter umfasst eine Positioniervorrichtung in Form eines Stabs, die in eine bereits bestehende Bohrung eingesteckt wird, und einen fahnenartigen Anzeigeabschnitt, dessen horizontale Länge den Abstand zwischen zwei Implantatkavitäten festlegt. Das freie Ende des Anzeigeabschnitts ist als rillenförmige Stütze für das Bohrwerkzeug des Dentalinstruments ausgeführt.

Nachteil dieses Abstandhalters ist, dass die horizontale Länge des Anzeigeabschnitts ausschließlich auf die Abmessungen der Implantate abgestimmt ist. Eine Berücksichtigung der medizinisch-biologischen Anforderungen des Knochengewebes des Kiefers wird durch diesen Abstandhalter nicht gewährleistet. Es besteht somit die Gefahr, dass die mit diesem Abstandhalter gesetzten Implantate durch Rückbildung des Knochengewebes locker werden, wodurch unerwünschte Nachbehandlungen notwendig werden sowie die Gefahr besteht, dass das Setzen von neuen Implantaten unmöglich wird.

Der vorliegenden Erfindung liegt daher die Aufgabe zugrunde einen Abstandhalter, der auch die medizinisch-biologischen Anforderungen des Knochengewebes des Kiefers berücksichtigt, sowie ein Herstellungsverfahren für einen derartigen Abstandhalter zu schaffen.

Ein weiterer Nachteil des Abstandhalters der EP '804 ist, dass der Abstandhalter erst eingesetzt werden kann, wenn bereits eine erste Bohrung durch den Zahnarzt gesetzt wurde. Die erste Bohrung muss entweder freihändig oder mit einer aufwendig herzustellenden Schablone durchgeführt werden.

Der vorliegenden Erfindung liegt daher auch die Aufgabe zugrunde einen Abstandhalter, der zum Bohren einer ersten Bohrung unter Berücksichtigung der Abmessungen des darin zu fixierenden Implantats und des benachbarten Zahns sowie der medizinisch-biologischen Anforderungen des Knochengewebes einsetzbar ist, und ein Herstellungsverfahren für einen derartigen Abstandhalter zu schaffen.

Diese Aufgaben werden gemäß der vorliegenden Erfindung durch einen Abstandhalter mit den Merkmalen des Anspruchs 1 und ein Herstellungsverfahren mit den Merkmalen des Anspruchs 30 gelöst. Besonders vorteilhafte Ausführungsformen sind in den Unteransprüchen 2 - 12 angeführt.

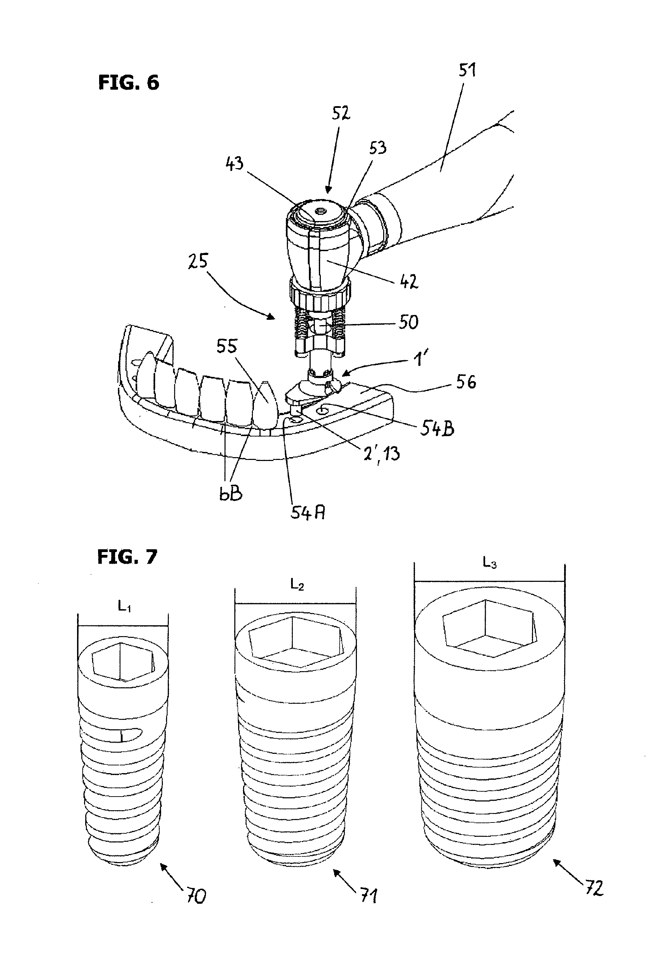

Bei dem erfindungsgemäßen Abstandhalter nach Anspruch 1 ist der Abstand zwischen der Positioniervorrichtung und der Stützvorrichtung für das Bohrwerkzeug (den Dentalbohrer) eine Funktion der Abmessung, d.h. des oder der Durchmesser, der zu setzenden Implantate, und eine Funktion der biologischen Breite, deren Wert zwischen 1,4 - 3,2 mm liegt, wodurch ein zuverlässigerer und natürlicherer Sitz der Implantate und des Zahnersatzes erreicht wird. Unter der biologischen Breite wird der Abstand zwischen zwei Zähnen bzw. Zahnersätzen bzw. zwischen einem Zahn und einem Zahnersatz auf der Basis des Kiefers, auf dem die Zähne oder Zahnersätze sitzen, verstanden (siehe Figur 6, wo die biologische Breite mit bB bezeichnet ist). In diesem Bereich zwischen den Zähnen bzw. Zahnersätzen bildet der Kieferknochen einen Kamm, der sich etwas über den restlichen Kieferknochen erhebt, so dass jeder Zahn in einem eigenen Bett, das durch die Kämme an den Seiten des Zahns gebildet wird, sitzt. Über diese Kämme wächst Mundschleimhaut und formt die so genannte Papille.

Die biologische Breite ist beim Setzen von Implantaten unbedingt zu berücksichtigen, um zu gewährleisten, dass der Kieferknochen zwischen den Zahnersätzen (Implantaten, Kronen) bzw. zwischen einem gesunden Zahn und dem Zahnersatz erhalten bleibt. Wird die biologische Breite beim Bohren der Implantatkavitäten nicht berücksichtigt, d.h. werden die Zahnersätze zu eng gesetzt, so besteht die Gefahr einer Unterversorgung des Kieferknochens in diesem Bereich, so dass Knochengewebe abgebaut wird. Die Folge ist ein schlechter Sitz und eine Lockerung des Zahnersatzes. Unterschreitet die biologische Breite einen Wert von 1,4 mm, nimmt die Gefahr der Unterversorgung des Kamms des Kieferknochens merkbar zu.

Erfindungsgemäß ist zwischen zwei Ausführungsformen des Abstandhalters zu unterscheiden: Eine erste Ausführungsform dient zum Bestimmen der Position einer Implantatkavität, die einem lebenden Zahn oder einem bereits am Kiefer befestigten, fertigen Zahnersatz benachbart ist. Der Anwender muss in einem ersten Schritt festlegen, wie groß der Durchmesser jenes Implantats ist, das er anschließend an den Zahn oder den Zahnersatz setzen möchte, und dann den passenden erfindungsgemäßen Abstandhalter auswählen. Diesen Abstandhalter positioniert er mit Hilfe der Positioniervorrichtung anschließend so auf jenem Kieferabschnitt, auf dem er die Implantatkavität zu bohren beabsichtigt, dass der Abstandhalter den Zahn bzw. den Zahnersatz kontaktiert. Sobald der Abstandhalter auf dem Kiefer positioniert ist, zeigt ihm die Stützvorrichtung für das Bohrwerkzeug jene Stelle am Kiefer an, an der die Implantatkavität zu bohren ist, wobei aufgrund des voreingestellten und feststehenden, d.h. nicht veränderbaren, Abstands zwischen der Positioniervorrichtung und der Stützvorrichtung die biologische Breite bereits mitberücksichtigt ist. In vorteilhafter Weise braucht der Anwender somit beim Bestimmen der Stelle, an der die Implantatkavität zu bohren ist, die biologische Breite nicht selbständig berücksichtigen bzw. ist die Gefahr, dass er vergisst, die biologische Breite zu berücksichtigen, eliminiert. Zusätzlich befindet sich die Stützvorrichtung exakt an jener Stelle, wo das Bohrwerkzeug zum Bohren der implantatkavität angesetzt wird, so dass eine sichere Abstützung und Führung des Bohrwerkzeugs beim Ansetzen auf dem Kiefer und während des Bohrvorganges gewährleistet ist.

Bei dieser ersten Ausführungsform des Abstandhalters entspricht der Abstand D1 zwischen der Positioniervorrichtung und der Stützvorrichtung der Gleichung D1 = L_{(1,2,3)}/2 + bB, wobei L_{(1,2,3)} gleich dem Durchmessers jenes Implantats ist, das in die Implantatkavität, die unter Zuhilfenahme des Abstandhalters herstellbar ist, einsetzbar ist, und bB gleich der biologischen Breite von 1,4 - 3,2 mm, bevorzugt 1,5 - 2,1 mm, besonders bevorzugt 1,8 - 2,0 mm ist. In Figur 7 sind zum besseren Verständnis drei Implantate dargestellt, wobei jedes Implantat einen eigenen Durchmesser L₁, L₂, L₃ hat.

Eine zweite Ausführungsform dient zum Bestimmen des Abstands zwischen einer ersten, bereits gebohrten Implantatkavität und einer zweiten, benachbarten, zu bohrenden Implantatkavität, wobei in beide Implantatkavitäten noch kein Zahnersatz angeordnet ist. Wiederum muss der Anwender vor Anwendung des Abstandhalters festlegen, welche Durchmesser die beiden Implantate haben, die in die Implantatkavitäten eingesetzt werden sollen und den passenden erfindungsgemäßen Abstandhalter auswählen. Diesen ausgewählten Abstandhalter positioniert er mit Hilfe der Positioniervorrichtung an der ersten Implantatkavität, so dass die Stützvorrichtung für das Bohrwerkzeug jene Stelle am Kiefer anzeigt, an der die zweite Implantatkavität zu bohren ist. Aufgrund des voreingestellten und feststehenden, d.h. nicht veränderbaren, Abstands zwischen der Positioniervorrichtung und der Stützvorrichtung ist die biologische Breite wiederum mitberücksichtigt.

Bei dieser zweiten Ausführungsform des Abstandhalters entspricht der Abstand D2 zwischen der Positioniervorrichtung und der Stützvorrichtung der Gleichung D2 = L_{(1,2,3)}/2 + L_{(1,2,3)}/2 + bB ist, wobei L_{(1,2,3)} gleich dem Durchmesser eines ersten und zweiten Implantats ist, die in die erste und zweite Implantatkavität, die nebeneinander angeordnet sind, einsetzbar sind, wobei zumindest eine Implantatkavität unter Verwendung des Abstandhalters herstellbar ist. bB ist die biologische Breite von 1,4 - 3,2 mm, bevorzugt 2,0 - 3,2 mm, besonders bevorzugt 2,5 - 3,0 mm.

Die Stützvorrichtung für das Bohrwerkzeug kann bei beiden Ausführungsformen des Abstandhalters durch jede bekannte Vorrichtung gebildet sein, beispielsweise durch eine Rille an dem freien Ende des Abstandhalters. Bevorzugt ist sie jedoch als Durchstich oder als hülsenförmiger Fortsatz ausgebildet, wodurch die Stütz- und Führungswirkung für das Bohrwerkzeug verstärkt wird.

Die Positioniervorrichtung der zweiten Ausführungsform des Abstandhalters kann jede beliebige geometrische Form und Ausführung aufweisen, die in der ersten Implantatkavität aufnehmbar ist, beispielsweise als rund oder eckig ausgebildeter Fortsatz oder als Hülse. Bevorzugt ist sie als Stift ausgebildet, der in etwa in das obere Drittel der ersten Implantatkavität ragt und somit eine sichere, rutschfeste Positionierung des Abstandhalters ermöglicht.

Der Abstandhalter gewährleistet im Vergleich zu bekannten Abstandhaltern eine reproduzierbare, auf verschiedene Implantate abgestimmte Anwendung unter Berücksichtigung der biologischen Breite.

Das Verfahren zur Herstellung eines erfindungsgemäßen Abstandhalters bei dem der Abstand D1, D2 zwischen dem Durchstich für das Bohrwerkzeug und der Positioniervorrichtung feststehend ist, besteht aus folgenden Schritten:
- Bestimmen des Abstands D1 gemäß dem Zusammenhang D1 = L_{(1,2,3)}/2 + bB oder Bestimmen des Abstands D2 gemäß dem Zusammenhang D2 = L_{(1,2,3)}/2 + L_{(1,2,3)}/2 + bB, wobei L_{(1,2,3)} gleich dem Durchmesser eines ersten bzw. eines ersten und zweiten Implantats entspricht und der Wert der biologischen Breite bB zwischen 1,4 - 3,2 mm liegt,
- Zur Verfügung stellen eines Werkstücks,
- Spanabhebende, bevorzugt fräsende, Bearbeitung des Werkstücks, so dass die Positioniervorrichtung im berechneten Abstand D1 oder D2 von der Stützvorrichtung angeordnet ist.

Ein derartiges Verfahren ermöglicht eine einfache und kostengünstige Herstellung aus einem einzigen Werkstück. Als Material des Werkstücks wird bevorzugt rostfreier, verchromter Stahl oder Titan verwendet, so dass der Abstandhalter bei mehrmaliger Verwendung auch gegenüber Reinigung und Sterilisation beständig ist. Alternativ kann die Herstellung des Abstandhalters durch Metallpulverspritzguss (MIM-Verfahren) erfolgen.

Ein weiterer Nachteil des Abstandhalters der EP '804 ist, dass die rillenförmige Stützvorrichtung keine ausreichende Stützung Führung für das Bohrwerkzeug des Dentalinstruments bietet, so dass die Gefahr besteht, dass die zu bohrende Implantatkavität nicht die gewünschte Richtung oder Parallelität zu ersten Kavität aufweist. Insbesondere ist das Ansetzten und Positionieren des Bohrwerkzeugs in die Stützrille in der räumlich beengten und zum Teil schlecht einsehbaren Mundhöhle schwierig.

Der vorliegenden Erfindung liegt somit des Weiteren die Aufgabe zugrunde eine implantologische Führungseinrichtung zu schaffen, die dem Anwender ein verbessertes Ansetzen und Positionieren des Dentalinstruments vor dem Bohrvorgang und eine zuverlässigere Führung und Stützung des Werkzeugs und des Dentalinstruments während des Bohrvorgangs gewährleistet.

Diese Aufgabe wird gemäß der vorliegenden Erfindung durch eine implantologische, insbesondere kieferchirurgische Führungseinrichtung mit den Merkmalen der Ansprüche 13 - 27 gelöst. Bevorzugt ist die implantologische Führungseinrichtung dabei mit einem erfindungsgemäßen Abstandhalter nach einem der Ansprüche 1 -12 bzw. mit einer Verbindungsvorrichtung zum Anschluss eines erfindungsgemäßen Abstandhalters nach Anspruch 1 - 12 versehen. Selbstverständlich kann die erfindungsgemäße implantologische Führungseinrichtung jedoch auch mit anderen, bekannten Abstandhaltern verbunden sein bzw. entsprechende Verbindungsvorrichtungen zum Anschluss dieser bekannten Abstandhalter aufweisen.

Die Verbindungsvorrichtung zum Anschluss eines erfindungsgemäßen oder eines anderen Abstandhalters ist bevorzugt als lösbare Verbindungsvorrichtung ausgeführt, besonders bevorzugt als Schraub-, Steck-, Rast- oder Klemmverbindung, so dass der Anwender unterschiedliche Abstandhalter, bevorzugt die erste und zweite Ausführungsform des erfindungsgemäßen Abstandhalters, mit einer einzigen Führungseinrichtung verwenden kann.

Um eine möglichst zuverlässige Führung des Bohrwerkzeugs zu gewährleisten umfasst die Führungseinrichtung eine Führungshülse, die das Werkzeug aufnimmt und führt. Bevorzugt ist der Durchmesser der Führungshülse nur geringfügig größer als der Durchmesser des Bohrwerkzeugs, so dass die Gefahr des Auslenkens des Bohrwerkzeugs während der Behandlung minimal ist.

In einem bevorzugten Ausführungsbeispiel ist an der Führungshülse zumindest ein Führungsstift zum Führen des Dentalinstruments angeordnet. In einer ersten Ausführungsform wird das Dentalinstrument während des Bohrvorgangs entlang dieses zumindest einen Führungsstifts verschoben. Besonders bevorzugt sind mehrere Führungsstifte vorgesehen, die so angeordnet sind, dass zumindest ein Teil des zu führenden Dentalinstruments, insbesondere der Instrumentenkopf, zwischen den Führungsstiften einsetzbar ist, wodurch eine bessere Führung und Stützung erreicht wird. In einer alternativen Ausführungsform kann das Dentalinstrument mit dem zumindest einen Führungsstift relativ zur Führungshülse verschoben werden. Bevorzugt weist die Führungseinrichtung eine Schiebebuchse zur Aufnahme zumindest eines Teils des zu führenden Dentalinstruments auf, wobei die Schiebebuchse für jeden Führungsstift mit einer Bohrung versehen ist, in die ein Abschnitt eines Führungsstifts eingreift. Beim Bohren der Implantatkavität wird die Schiebebuchse mit dem darin aufgenommenen Dentalinstrument und mit dem zumindest einen Führungsstift oder entlang des zumindest eine Führungsstifts verschoben.

Der zumindest eine Führungsstifte ist bevorzugt an einem Lagerblock angeordnet, der mit der Führungshülse verbunden ist.

In einem besonders bevorzugten Ausführungsbeispiel ist eine Aufnahmevorrichtung für zumindest einen Teil des Dentalinstruments, bevorzugt den Instrumentenkopf, vorgesehen, die mit der Schiebebuchse verbunden ist. In vorteilhafter Weise ist die Aufnahmevorrichtung so ausgebildet, dass sie mit dem Dentalinstrument eine lösbare Verbindung eingeht, zum Beispiel als Schraub-, Steck-, Rast- oder Klemmverbindung, so dass die Führungseinrichtung fest mit dem Dentalinstrument verbunden ist. Der Anwender kann damit schon vor dem Beginn der Behandlung, außerhalb der Mundhöhle des Patienten die Führungseinrichtung und bevorzugt auch den Abstandhalter mit dem Dentalinstrument verbinden. Zum Start der Behandlung ist es dann nur noch notwendig, die zusammengesetzte Behandlungsvorrichtung, bestehend aus Dentalinstrument, Führungseinrichtung und Abstandhalter, auf dem Kiefer zu positionieren. Damit entfällt im Vergleich mit dem Stand der Technik das Ansetzten und Positionieren des Dentalinstruments und des Bohrwerkzeugs am Kiefer oder an der Führungseinrichtung in der räumlich beengten und schlecht einsehbaren Mundhöhle. Zur weiteren Erleichterung ist die Aufnahmevorrichtung gegenüber der Schiebebuchse drehbar ausgebildet, so dass der Anwender während der Behandlung das Dentalinstrument in Bezug auf die Führungseinrichtung verdrehen kann.

Die Erfindung wird nachfolgend anhand bevorzugter Ausführungsbeispiele und Bezug nehmend auf die beigefügten Zeichnungen erläutert:
Figuren 1A und 1 B zeigen in einer perspektivischen bzw. Schnittdarstellung eine erste Ausführungsform des erfindungsgemäßen Abstandhalters.
Figuren 2A und 2B zeigen in einer perspektivischen bzw. Schnittdarstellung eine zweite Ausführungsform des erfindungsgemäßen Abstandhalters.
Figur 3 zeigt ein Werkzeugset für Werkzeuge zum Bohren von Implantatkavitäten.
Figuren 4A und 4B zeigen in einer perspektivischen bzw. Schnittdarstellung die erfindungsgemäße implantologische Führungseinrichtung jeweils mit dem erfindungsgemäßen Abstandhalter.
Figur 5 zeigt ein Hilfsmittel zur Auswahl eines erfindungsgemäßen Abstandhalters in Form einer Tabelle.
Figur 6 zeigt die erfindungsgemäße implantologische Führungseinrichtung mit dem erfindungsgemäßen Abstandhalter angeschlossen an ein Dentalinstrument mit Werkzeug beim Bohren von Implantatkavitäten in einem Kiefer.
Figur 7 zeigt drei Implantat verschiedener Abmessungen.

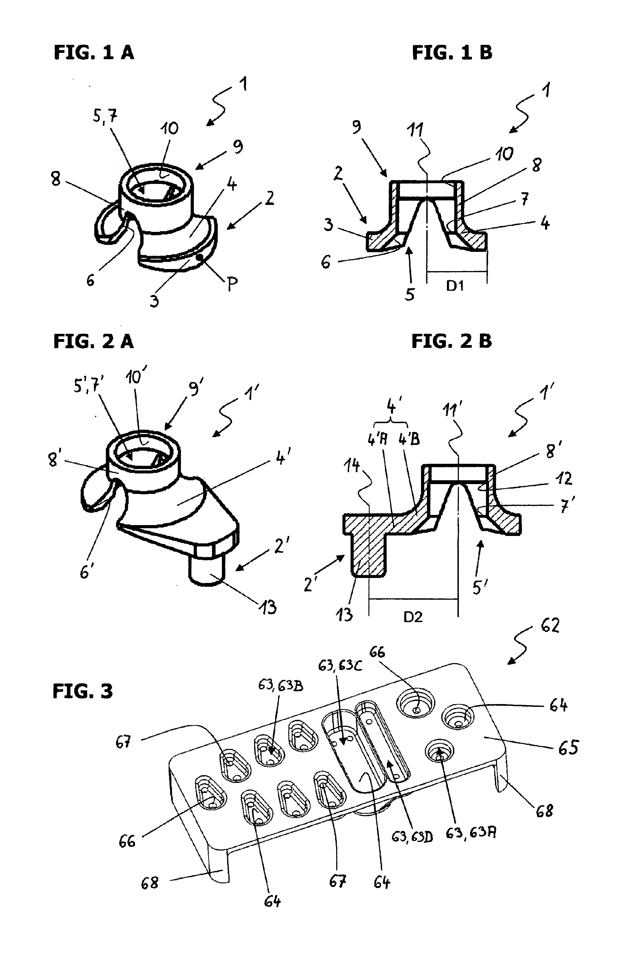

Der Abstandhalter 1 nach Figur 1A und 1B dient zum Bestimmen der Position einer Implantatkavität 54A (siehe Figur 6), die einem lebenden Zahn 55 oder einem bereits am Kiefer befestigten, fertigen Zahnersatz benachbart ist. Er umfasst eine Positioniervorrichtung 2, die als Schulter 3 ausgebildet ist, und einen Kontaktpunkt P, mit dem die Schulter 3, sobald der Abstandhalter 1 auf den Kieferabschnitt 56, in dem die Implantatkavität 54A zu bohren ist, gesetzt ist, einen Zahn 55 oder einen Zahnersatz kontaktiert, der an diesen Kieferabschnitt 56 anschließt. Kontaktpunkt P berührt den Zahn 55 bzw. den Zahnersatz dabei im Bereich des Zahnhalses jener Seite des Zahns 55 des Zahnersatzes, die dem Kieferabschnitt 56 am nächsten ist (der mesialen bzw. distalen Seite). Die Schulter 3 ist kreisförmig bzw. kreisbogenförmig ausgebildet, wobei der Kontaktpunkt P am Außenumfang des Kreises oder Kreisbogens angeordnet ist. Die Schulter 3 ist bevorzugt so geformt, dass sie an die Form des Zahnhalses des Zahns 55 angepasst ist, insbesondere hat sie eine Höhe von etwa 0,8 - 1,2 mm, bevorzugt 1,0 mm, so dass sie vom Anwender ideal an den benachbarten Zahn 55 oder Zahnersatz angelegt werden kann.

Über Steg 4 wird die Positioniervorrichtung 2 mit der Stützvorrichtung 5 für das Bohrwerkzeug 50 verbunden. Der Steg 4 und die Positioniervorrichtung 2 umfassen zumindest eine, bevorzugt zwei, Aussparungen 6, so dass Steg 4 und Positioniervorrichtung 2 in zwei kreisbogenförmige Abschnitte unterteilt sind. Die zumindest eine Aussparung 6 ermöglicht dem Anwender in das Innere des Abstandhalters 1 und der Stützvorrichtung 5 zu blicken und das Bohrwerkzeug 50 während des Bohrens der Implantatkavität 54A zu beobachten.

Bevorzugt ist auf dem Steg 4 eine Markierung angebracht, so dass der Anwender unterschiedliche Abstandhalter 1 einfach unterscheiden kann. Die Markierung kann durch ein oder mehrere alphanumerische Zeichen, Symbole oder einen Farbstoff gebildet sein.

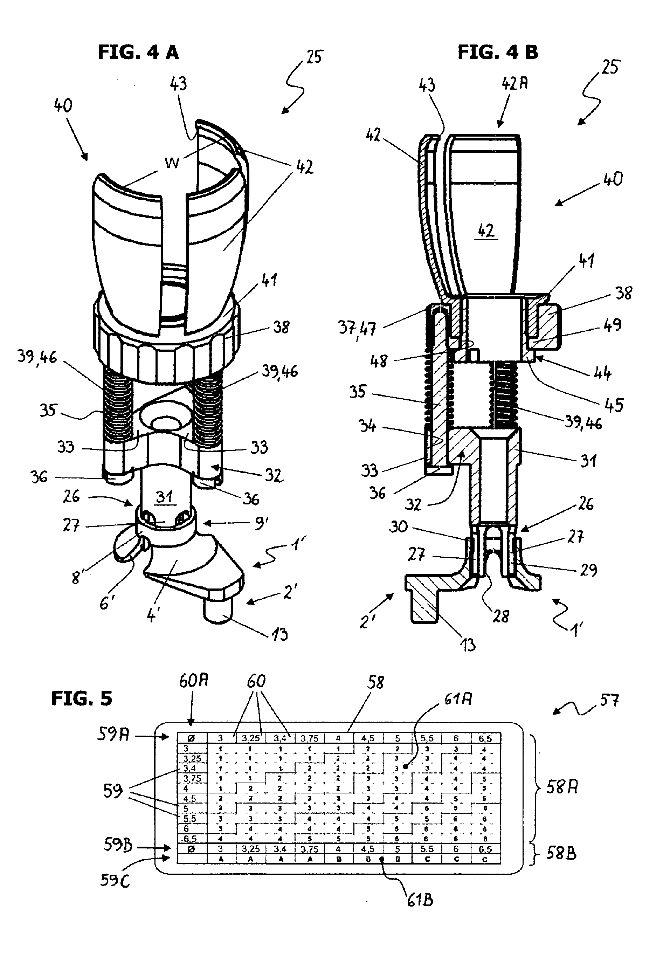

Die Stützvorrichtung 5 ist als kreisförmiger Durchstich 7 ausgebildet, sie kann jedoch auch andere geometrische Formen aufweisen. Der Durchmesser des Durchstichs 7 ist so bemessen, dass der Abstandhalter 1 mit allen bekannten Bohrwerkzeugen verwendet werden kann. An den Durchstich 7 schließt eine Anschlusshülse 8 an, die die Stützung und Führung des Bohrwerkzeugs 50 verbessert. Bevorzugt umfasst die Anschlusshülse 8 eine Verbindungsvorrichtung 9 zum Anschluss des Abstandhalters 1 an eine implantologische, insbesondere kieferchirurgische Führungseinrichtung, zum Beispiel Führungseinrichtung 25 (siehe Fig. 4A, 4B). Die Verbindungsvorrichtung 9 ist bevorzugt als lösbare Verbindungsvorrichtung, besonders bevorzugt als Schraub-, Steck-, Rast- oder Klemmverbindung, ausgebildet. In den Figuren 1A und 1B ist beispielhaft eine Schulter 10 für eine Steckverbindung abgebildet.

In Figur 1 B ist des Weiteren die Mittelachse 11 der Stützvorrichtung 5 dargestellt. Erfindungsgemäß entspricht der Abstand D1 zwischen der Mittelachse 11 und der Positioniervorrichtung 2, insbesondere dem Kontaktpunkt P der Schulter 3, der Summe des halben Durchmessers L₁ oder L₂ oder L₃ jenes Implantats 70 - 72 (siehe Figur 7), das in die Implantatkavität 54A, die unter Zuhilfenahme des Abstandhalters 1 herstellbar ist, eingesetzt werden soll, und der biologischen Breite bB, die einen Wert von 1,4 - 3,2 mm, bevorzugt 1,5 - 2,1 mm, besonders bevorzugt 1,8 - 2,0 mm hat (D1 = L_{(1,2,3)}/2 + bB).

Der Abstandhalter 1' nach Figur 2A und 2B dient zum Bestimmen des Abstands zwischen einer ersten, bereits gebohrten Implantatkavität 54A und einer zweiten, benachbarten, zu bohrenden Implantatkavität 54B (siehe Figur 6), wobei in beide Implantatkavitäten 54A, 54B noch kein Zahnersatz angeordnet ist. Die Positioniervorrichtung 2' ist als zylindrischer Stift 13 ausgebildet, dessen Durchmesser so gewählt ist, dass er vom Anwender in die erste, bereits gebohrte Implantatkavität 54A eingesteckt werden kann. Steg 4' zur Verbindung der Positioniervorrichtung 2' mit der Stützvorrichtung 5' umfasst einen ersten, im wesentlich horizontalen Stegabschnitt 4'A und einen zweiten, sich in Richtung Stützvorrichtung 5' verjüngenden Stegabschnitt 4'B. Auf einem der beiden Stegabschnitte 4'A und 4'B kann wiederum eine durch ein oder mehrere alphanumerische Zeichen, Symbole oder einen Farbstoff gebildete Markierung angeordnet sein.

Die übrigen Bauteile des Abstandhalters 1', i.e. Aussparung 6', Anschlusshülse 8', der die Stützvorrichtung 5' bildenden Durchstich 7' und die Verbindungsvorrichtung 9' entsprechen den Bauteilen des Abstandhalters 1. Schulter 10' von Verbindungsvorrichtung 9' ist hier beispielhaft als Teil einer Rastverbindung ausgestattet, an deren dem Steg 4' zugewandten Ende ein Rücksprung 12 vorgesehen ist, in den entsprechende Rastelemente (zum Beispiel Rastnase 29) der mit dem Abstandhalter 1' verbindbaren implantologischen Führungseinrichtung 25 eingreifen (siehe Fig. 4A, 4B).

Figur 2B zeigt die Mittelachse 14 der Positioniervorrichtung 2 und die Mittelachse 11' der Stützvorrichtung 5'. Erfindungsgemäß entspricht der Abstand D2 zwischen der Mittelachse 11' und der Mittelachse 14 der Summe des halben Durchmessers L₁ oder L₂ oder L₃ jenes ersten und zweiten Implantats 70 - 72 (siehe Figur 7), das in die erste und zweite Implantatkavität 54A, 54B, die nebeneinander angeordnet sind, eingesetzt werden sollen, wobei zumindest eine Implantatkavität 54A, 54B unter Zuhilfenahme des Abstandhalters 1' herstellbar ist, und der biologischen Breite bB, die einen Wert von 1,4 - 3,2 mm, bevorzugt 2,0 - 3,2 mm, besonders bevorzugt 2,5 - 3,0 mm hat (D2 = L_{(1,2,3)}/2 + L_{(1,2,3)}/2 + bB).

Die Figuren 4A und 4B zeigen eine erfindungsgemäße implantologische, insbesondere kieferchirurgische Führungseinrichtung 25, die mit einem erfindungsgemäßen Abstandhalter 1' verbunden ist. Anstelle des Abstandhalters 1' kann auch Abstandhalter 1 in gleicher Weise mit der Führungseinrichtung 25 verbunden sein. Selbstverständlich kann die erfindungsgemäße implantologische Führungseinrichtung 25 auch mit anderen Abstandhaltern verbunden sein und entsprechende Verbindungsvorrichtungen zum Anschluss dieser Abstandhalter aufweisen und umgekehrt der erfindungsgemäße Abstandhalter 1, 1' mit anderen Führungseinrichtungen verbindbar sein und mit entsprechenden Verbindungsvorrichtungen zum Anschluss an diese Führungseinrichtungen ausgestattet sein.

Führungseinrichtung 25 umfasst eine Verbindungseinrichtung 26, die hier beispielhaft als lösbare Rastverbindung ausgebildet ist. Verbindungseinrichtung 26 besteht aus zumindest einem Federarm, bevorzugt aus drei durch Einstiche 28 voneinander getrennten Federarmen 27. Jeder Federarm 27 trägt eine Rastnase 29 und einen Rücksprung 30. Bei der Verbindung der Führungseinrichtung 25 mit einem Abstandhalter, zum Beispiel Abstandhalter 1', hintergreifen die Rastnasen 29 Schulter 10' der Verbindungsvorrichtung 9' des Abstandhalters 1' und rasten in den an die Schulter 10' anschließenden Rücksprung 12 ein. In gleicher Weise rastet Schulter 10' in den Rücksprung 30 des zumindest einen Federarms 27 ein. Um eine für den Anwender leichtgängige Kupplung des Abstandhalters 1' an die Führungseinrichtung 25 und gleichzeitig eine verlässliche, nicht zu leicht lösbare Verbindung zu gewährleisten, ist der zumindest eine Federarm 27 leicht nach außen vorgespannt, jedoch radial nach innen federnd ausgebildet, so dass während des Kupplungsvorgangs ein aneinander Vorbeischieben der Rastnase 29 an der Schulter 10' ohne zu großen Kraftaufwand möglich ist.

Einstich 28 und Aussparung 6' des Abstandhalters 1'sind in gekuppeltem Zustand bevorzugt übereinander angeordnet, so dass der Anwender eine bestmögliche Sicht auf das Bohrwerkzeug 50 hat.

Anschließend an Verbindungsvorrichtung 26 sind Führungsmittel, bevorzugt eine Führungshülse 31, angeordnet, die das Bohrwerkzeug 50 aufnehmen, führen und stützen. Bevorzugt ist der Innendurchmesser der Führungshülse 31 mit etwa 2,05 - 2,20 mm nur geringfügig größer als der Durchmesser des Bohrwerkzeugs 50, so dass die Führungshülse 31 insbesondere eine gute Stützwirkung garantiert. An der Führungshülse 31 ist zumindest ein Führungsstift 35 angeordnet, bevorzugt sind drei Führungsstifte 35 vorgesehen. Die Führungsstifte 35 dienen der Führung des Dentalinstruments 51 während des Bohrens der Implantatkavität 54A, 54B, bevorzugt führen sie den Instrumentenkopf 52 des Dentalinstruments 51 (siehe Figur 6).

An dem der Verbindungsvorrichtung 26 abgewandten Ende der Führungshülse 31 ist ein Lagerblock 32 angeordnet, dessen Durchmesser größer ist als der Durchmesser der Führungshülse 31. Lagerblock 32 umfasst einen oder mehrere Flügel 33, die mit jeweils zumindest einer Bohrung 34 versehen sind. Die Führungsstifte 35 sind in den Bohrungen 34 durch Gleitsitze aufgenommen und ragen mit einem ersten Ende, von der Führungshülse 31 wegweisend durch die Bohrungen 34. An ihrem zweiten Ende weisen die Führungsstifte 35 jeweils einen Anschlag 36 auf, dessen Durchmesser breiter ist als der Durchmesser der Stifte 35 und der Durchmesser der Bohrungen 34.

Ein Abschnitt des zumindest einen Führungsstifts 35 ist in einer Bohrung 37 einer Schiebebuchse 38 aufgenommen, bevorzugt darin verschraubt. Bohrung 37 ist bevorzugt als Sackloch 47 ausgebildet, so dass das erste Ende des zumindest einen Führungsstifts 35 darin aufgenommen ist und das Eindringen und Ablagern von Partikeln und Verschmutzungen möglichst verringert ist. Die Schiebebuchse 38 dient als Auflager zumindest eines Teils des zu führenden Dentalinstruments 51, bevorzugt des Instrumenten kopfs 52, und umfasst eine Bohrung 48, durch die Bohrwerkzeug 50 hindurch treten kann. Ist ein Dentalinstrument 51 auf der Schiebebuchse 38 gelagert und wird, zum Beispiel während des Bohrens der Implantatkavität, durch das Dentalinstrument 51 eine Kraft auf die Schiebebuchse 38 ausgeübt, so bewegt sich die Schiebebuchse 38 mit den Führungsstiften 35 in Richtung der Führungshülse 31, wobei die Führungsstifte 35 durch die Bohrungen 34 im Lagerblock 32 gleiten.

Bevorzugt ist zwischen der Führungshülse 31 bzw. dem Lagerblock 32 und der Schiebebuchse 38 zumindest ein Federelement 39 angeordnet, das die Schiebebuchse 38 gegen die Führungshülse 31 vorspannt. Bevorzugt sind mehrere Federelemente 39 vorgesehen, die besonders bevorzugt als Spiralfedern 46 ausgebildet sind, wobei jeweils ein Führungsstift 35 im Inneren der Spiralfeder 46 angeordnet ist. Der Durchmesser der Bohrungen 34 des Lagerblocks 32 ist geringer als der Durchmesser des Federelements 39, so dass das Federelement 39 auf der der Schiebebuchse 38 zugewandten Seite des Lagerblocks 32 aufsitzt. Das zumindest eine Federelement 39 bewirkt in vorteilhafter Weise, dass die Schiebebuchse 38 der Führungseinrichtung 25 in unbelastetem Zustand, zum Beispiel vor Beginn des Bohrvorgangs der Implantatkavität 54A, 54B, immer den größtmöglichen Abstand von der Führungshülse 31 aufweist, wobei der Abstand so bemessen ist, dass, wenn die Führungseinrichtung 25 mit einem Abstandhalter 1, 1' und einem Dentalinstrument 51, in das ein Bohrwerkzeug 50 eingespannt ist, verbunden ist, die Spitze des Bohrwerkzeugs 50 nicht oder nur sehr gering über den Abstandhalter 1, 1' hinausragt. Das ermöglicht dem Anwender ein einfaches Hantieren mit dem Abstandhalter 1, 1' und ein gefahrloses Positionieren des Abstandhalters 1, 1' auf dem Kiefer 56. Wirkt auf die Schiebebuchse 38 eine Druckkraft ein, so muss die Schiebebuchse 38 mit dem zumindest einen Führungsstift 35 gegen die Federkraft des zumindest einen Federelements 39 in Richtung der Führungshülse 31 bewegt werden. Sobald die Kraftausübung auf die Schiebebuchse 38 beendet ist, nimmt die Schiebebuchse 38 selbständig wieder den größtmöglichen Abstand von der Führungshülse 31 ein. Zusätzlich wirkt das Federelement 39 als Anschlag, das, wenn es vollständig komprimiert ist, die Eindringtiefe des Bohrwerkzeugs 50 begrenzt und ein zu tiefes Bohren in den Kieferabschnitt 56 verhindert.

Bevorzugt ist der Außenumfang der Schiebebuchse 38 mit einem Griffmuster versehen, um das Hantieren mit der Führungseinrichtung 25 und insbesondere den Anschluss an das Dentalinstrument 51 für den Anwender zu erleichtern.

Mit der Schiebebuchse 38 verbunden ist eine Aufnahmevorrichtung 40 für zumindest einen Teil des Dentalinstruments 51, bevorzugt den Instrumentenkopf 52. In einem bevorzugten Ausführungsbeispiel ist die Aufnahmevorrichtung 40 gegenüber der Schiebebuchse 38 drehbar angeordnet, so dass der Anwender beim Positionieren des Abstandhalters 1, 1' auf dem Kieferabschnitt 56 und während der Behandlung das Dentalinstrument 51 in Bezug auf die Führungseinrichtung 25 und den Abstandhalter 1, 1' verdrehen kann. Dazu ist eine Ringhülse 44 vorgesehen, die durch Bohrung 48 von Schiebebuchse 38 ragt, wobei Flansch 45 der Ringhülse 44 an der der Führungshülse 31 zugewandten Seite der Schiebebuchse 38 anliegt. Ringhülse 44 ist mit Tragehülse 41 der Aufnahmevorrichtung 40 zum Beispiel durch Verschrauben oder Verkleben so verbunden, dass zwischen dem Flansch 45 und der Tragehülse 41 die Basisfläche 49 der Schiebebuchse 38 gleitend aufgenommen ist.

Bevorzugt besteht die Aufnahmevorrichtung 40 neben der zylindrischen Tragehülse 41 aus zumindest zwei federnden Flügeln 42. Die lichte Weite W zwischen den zumindest zwei federnden Flügeln 42 ist so bemessen, dass dazwischen der Instrumentenkopf 52 einsteckbar ist, wobei die lichte Weite W geringer als der Durchmesser des Instrumentenkopfs 52 ist, so dass bei Einstecken des Instrumentenkopfs 52 in die Aufnahmevorrichtung 40 die Flügel 42 radial nach außen federnd und aufgrund ihrer konkaven Form zumindest teilweise am Instrumentenkopf 52 anliegend eine lösbare Verbindung mit dem Instrumentenkopf 52 eingehen. Besonders bevorzugt hat das freie Ende 42A zumindest eines Flügels 42 eine Rastkante 43, die, wenn der Instrumentenkopf 52 in die Aufnahmevorrichtung 40 eingesteckt ist, in einen Rücksprung 53 am Instrumentenkopf 52 eingreift. In vorteilhafter Weise kann durch die Aufnahmevorrichtung 40 die Führungseinrichtung 25 und der Abstandhalter 1, 1' am Dentalinstrument 51 befestigt und die gesamte Behandlungsvorrichtung, bestehend aus dem Dentalinstrument 51, der Führungseinrichtung 25 und dem Abstandhalter 1, 1' in einfacher Weise auf dem Kiefer 56 positionieren werden, so wie es beispielhaft in Figur 6 dargestellt ist.

Neben der Behandlungsvorrichtung, bestehend aus dem Dentalinstrument 51, der Führungseinrichtung 25 und dem Abstandhalter 1', ist in Figur 6 des Weiteren ein Kieferabschnitt 56 abgebildet, in dem mehrere Implantatkavitäten zu setzen sind. Eine erste, einem lebenden Zahn 55 benachbarte Implantatkavität 54A wurde bereits gebohrt, bevorzugt mit einem erfindungsgemäßen Abstandhalter 1 (siehe Figur 1, 1A). Mit Hilfe des erfindungsgemäßen Abstandhalters 1' wurde soeben eine zweite, der ersten Implantatkavität 54A benachbarte Implantatkavität 54B gebohrt. Positioniervorrichtung 2' in Form von Stift 13 wurde dabei in die erste Implantatkavität 54A gesteckt, wodurch der gewählte Abstand zur noch zu bohrenden Implantatkavität 54B festgelegt wurde. In gleicher Weise kann zumindest eine weitere Implantatkavität im Kieferabschnitt 56 gebohrt werden, wobei der Anwender, abhängig von dem zu setzenden Implantat 70 - 72, den selben Abstandhalter 1' oder einen Abstandhalter mit einem anderen Abstand zwischen der Positioniervorrichtung 2' und der Stützvorrichtung 5' verwenden. In diesem Fall würde der Anwender den Abstandhalter 1' von der Führungseinrichtung 25 lösen und einen anderen Abstandhalter mit der Führungseinrichtung 25 verbinden.

Die Entscheidung, welcher Abstandhalter 1, 1' zu verwenden ist, trifft der Anwender vor dem eigentlichen Bohrvorgang in Abhängigkeit des Implantats oder der Implantate 70 - 72, das/die in den Kieferabschnitt 56 gesetzt werden soll(en). Die Auswahl des Implantats hängt unter anderem von der Form sowie der Knochenstärke des Kieferabschnitts 56 und dem zu implantierenden Zahnersatz ab. Plant der Anwender zum Beispiel in Implantatkavität 54A Implantat 71 mit Durchmesser L₂ zu setzen, so muss er dafür jenen Abstandhalter 1 wählen, dessen Abstand D1 zwischen der Stützvorrichtung 5 und der Positioniervorrichtung 2 gleich D1 = L₂/2 + bB ist. Entsprechend ist das Vorgehen, wenn der Anwender Implantat 70 mit Durchmesser L₁ oder Implantat 72 mit Durchmesser L₃ verwenden möchte.

Beim anschließenden Bohren der Implantatkavität 54B muss der Anwender wiederum entscheiden, welches Implantat 70 - 72 er in die Kavität 54B einbringt. Wählt er beispielhaft Implantat 72 mit Durchmesser L₃, dann muss er jenen Abstandhalter 1' wählen, dessen Abstand D2 zwischen der Stützvorrichtung 5' und der Positioniervorrichtung 2' gleich D2 = L₂/2 + L₃/2 + bB ist, wobei der Term L₂/2 aus dem zuvor gewählten Implantat 71 mit dem Durchmesser L₂ der benachbarten Implantatkavität 54A resultiert. Entsprechend geht der Anwender vor, wenn er eine weitere, nicht dargestellte Implantatkavität in Kieferabschnitt 56 bohren möchte, wobei der Abstand D2 des zu verwendenden Abstandhalters 1' gleich D2 = L₃/2 + L_{(1,2,3)}/2 + bB ist, der Term L₃/2 aus dem zuvor gewählten Implantat 72 mit dem Durchmesser L₃ der Implantatkavität 54B resultiert und der Term L_{(1,2,3)}/2 für das in die nicht dargestellte Implantatkavität zu setzende und vom Anwender noch auszuwählende Implantat 70, 71 oder 72 steht. Selbstverständlich gibt es mehr als drei Implantate mit unterschiedlichen Durchmessern, so dass die Implantate 70 - 72 in Figur 7 nur exemplarisch zur Erläuterung der Vorgehensweise dargestellt sind, wobei das grundlegende Prinzip der Auswahl des Abstandhalters 1, 1' in Abhängigkeit vom Durchmesser des jeweiligen Implantats/der jeweiligen Implantate für alle bekannten dentalen Implantate anwendbar ist.

Aus der vorhergehenden Beschreibung folgt, dass für jede mögliche Anordnung zweier Implantate mit gleichen oder unterschiedlichen Durchmessern in benachbarten Implantatkavitäten 54A, 54B ein eigener Abstandhalter 1' mit dem entsprechenden Abstand D2 zwischen der Stützvorrichtung 5' und der Positioniervorrichtung 2' vorzusehen ist. Aufgrund der bereits erwähnten großen Anzahl von Implantaten, die von verschiedenen Herstellern angeboten werden, bedeutet dies, dass eine sehr große Anzahl von Abstandhaltern 1' für den Anwender zur Verfügung zu stellen ist. In einem bevorzugten Ausführungsbeispiel wird die Anzahl der unterschiedlichen Abstandhalter 1' auf ein für den Hersteller und Anwender zweckdienliche Anzahl reduziert, in dem die natürliche Variabilität der biologischen Breite bB genutzt wird. Natürlichen, biologischen Systemen entsprechend ist die biologische Breite bB zwischen gesunden Zähnen kein konstanter Faktor, sondern ist ihre Abmessung abhängig vom Zahntyp (Schneidezähne, Backenzähne, etc.), von der Kiefergröße, von der Zahnstellung etc., wobei die biologische Breite bB üblicherweise in einem Bereich von 1,4 - 3,2 mm liegt. Daraus folgt, dass ein Abstandhalter 1' mit einem feststehenden, unveränderlichen Abstand D2 zwischen der Stützvorrichtung 5' und der Positioniervorrichtung 2' für mehrere Kombinationen von gleichen oder unterschiedlichen Implantaten verwendet werden kann, wobei die biologische Breite bB in Abhängigkeit der Durchmesser der verwendeten Implantate innerhalb des Bereich von 1,4 - 3,2 mm variiert.

Figur 5 zeigt ein Hilfsmittel 57 zur Auswahl eines Abstandhalters 1 oder 1' für den Anwender, bevorzugt in Form einer Tabelle 58, an Hand dessen die Verwendung eines Abstandhalters 1' für mehrere Kombinationen von gleichen oder unterschiedlichen Implantaten erläutert wird: Tabelle 58 besteht aus einer ersten Untertabelle 58A für Abstandhalter 1' und einer zweiten Untertabelle 58B für Abstandhalter 1. Untertabelle 58A umfasst mehrere Zeilen 59 und mehrere Spalten 60. In der obersten Zeile 59A und in der ersten Spalte 60A sind die Durchmesser verschiedener Implantate, zum Beispiel auch der Implantate 70 - 72, angeführt. Die in Zeile 59A angeführten Werte (oder alternativ die in Spalte 60A angeführten Werte) geben den lmplantatdurchmesser in Millimetern für die erste, bereits gebohrte Implantatkavität 54A an, entsprechen also dem ersten Term L_{(1,2,3)}/2 der Gleichung D2 = L_{(1,2,3}/2 + L_{(1,2,3)}/2 + bB. Die in Spalte 60A angeführten Werte (oder alternativ die in Zeile 59A angeführten Werte) geben den Implantatdurchmesser in Millimetern für die zweite, noch zu bohrende Implantatkavität 54B an, entsprechen also dem zweiten Term L_{(1,2,3)}/2 dieser Gleichung. Die in der Ergebnismatrix 61A angeführten Ziffern 1 - 6 entsprechen sechs unterschiedlichen Abstandhaltern 1', die auch auf dem jeweiligen Abstandhalter 1', bevorzugt auf dessen Steg 4', angeordnet sind und dem Anwender das Erkennen des über das Hilfsmittel 57 ermittelten Abstandhalters 1' erleichtern.

Zur Auswahl des passenden Abstandhalters 1' sucht der Anwender in der Zeile 59A den Durchmesser des Implantats, das er in die Implantatkavität 54A setzen wird. Dann sucht er in der Spalte 60A den Durchmesser des Implantats, das er in die Implantatkavität 54B setzen wird. Anschließend bestimmt er jene Zelle der Ergebnismatrix 61A, in der sich die Spalte, die auf den Durchmesser des Implantats für die Implantatkavität 54A folgt, und die Zeile, die sich an den Durchmesser des Implantats für die Implantatkavität 54B anschließt, treffen. Die in dieser Zelle angeführte Ziffer gibt dem Anwender den zu verwendenden Abstandhalter 1' an.

Möchte der Anwender zwei Implantate nebeneinander setzen, die beide jeweils einen Durchmesser von 3 mm haben, so zeigt Hilfsmittel 57 die Verwendung des Abstandhalters 1' mit der Ziffer 1 an. Der Abstand D2 zwischen der Stützvorrichtung 5' und der Positioniervorrichtung 2' (Stift 13) des Abstandhalters 1' mit der Ziffer 1 beträgt zum Beispiel 6,1 mm. Gemäß der Gleichung D2 = L_{(1,2,3)}/2 + L_{(1,2,3)}/2 + bB ergibt sich damit:
6,1 mm = 3/2 mm + 3/2 mm + bB, womit die biologische Breite bB 3,1 mm beträgt. Möchte der Anwender zwei Implantate nebeneinander setzen, wobei das erste Implantat einen Durchmesser von 3 mm und das zweite Implantat einen Durchmesser von 4 mm hat, so zeigt Hilfsmittel 57 ebenfalls die Verwendung des Abstandhalters 1' mit der Ziffer 1 an. Gemäß der Gleichung D2 = L(_{1,2,3})/2 + L_{(1,2,3)}/2 + bB ergibt sich damit:
6,1 mm = 3/2 mm + 4/2 mm + bB, womit die biologische Breite bB 2,6 mm beträgt. In beiden Fällen liegt der Wert der biologischen Breite bB damit im gewünschten Bereich von 1,4 - 3,2 mm. In gleicher Weise variiert die biologische Breite bB auch bei der Verwendung der Abstandhalter 1' mit den Ziffern 2 - 6 und unterschiedlichen Implantaten. Durch Variation der biologischen Breite innerhalb der Grenzen von 1,4 - 3,2 mm wird erreicht, dass unter Berücksichtigung der medizinisch-biologischen Anforderungen des Knochengewebes des Kiefers die Anzahl der zu verwendenden Abstandhalter 1' in für Anwender und Hersteller vernünftigen Grenzen bleibt.

Untertabelle 58B dient zur Auswahl des passenden Abstandhalters 1 zum Bestimmen der Position einer Implantatkavität 54A, die einem lebenden Zahn 55 oder einem bereits am Kiefer befestigten, fertigen Zahnersatz benachbart ist. Untertabelle 58B besteht aus einer ersten Zeile 59B, in der der Implantatdurchmesser in Millimetern des Implantats, das in die zu bohrende Implantatkavität 54A eingesetzt werden soll, angegeben ist. Zeile 59C gibt die Ergebnismatrix 61 B wider, wobei die verschiedenen Abstandhalter 1 durch Buchstaben A - C unterschieden werden. Jeder Abstandhalter 1 kann wiederum für mehrere Implantate mit unterschiedlichen Durchmessern verwendet werden, wobei die biologische Breite bB variiert. Als Beispiel sei Abstandhalter 1 mit der Markierung A angeführt: Der Abstand D1 zwischen der Stützvorrichtung 5 und der Positioniervorrichtung 2 entspricht der Gleichung D1 = L_{(1,2,3)}/2 + bB und beträgt 3,8 mm. Für ein Implantat mit dem Durchmesser 3,0 mm ergibt sich damit:
3,8 mm = 3/2 mm + bB und für die biologische Breite bB ein Wert von 2,3 mm.
Für ein Implantat mit dem Durchmesser 3,75 mm, für das gemäß Untertabelle 58B ebenfalls Abstandhalter 1 mit der Markierung A zu verwenden ist, ergibt sich:
3,8 mm = 3,75/2 mm + bB und für die biologische Breite bB ein Wert von 1,925 mm. In beiden Fällen liegt die biologische Breite bB damit im gewünschten Bereich von 1,4 - 3,2 mm und es ist wie beim Abstandhalter 1' gewährleistet, dass unter Berücksichtigung der medizinisch-biologischen Anforderungen des Knochengewebes des Kiefers die Anzahl der zu verwendenden Abstandhalter 1 in für Anwender und Hersteller vernünftigen Grenzen bleibt.

Anstelle der dargestellten Ziffern oder Buchstaben in der Ergebnismatrix 61A, 61 B können die unterschiedlichen Abstandhalter im Hilfsmittel 57 auch durch andere Arten von Markierungen, zum Beispiel Symbole oder einen Farbstoff voneinander unterscheidbar gemacht sein.

In dem dargestellten Ausführungsbeispiel dient Hilfsmittel 57 zur Auswahl von drei unterschiedlichen Abstandhaltern 1 und sechs Abstandhaltern 1'. Hilfsmittel 57 ist jedoch nicht auf diese Anzahl von Abstandhaltern 1, 1' beschränkt, sondern es kann selbstverständlich auch für die Auswahl einer höheren oder geringeren Anzahl von Abstandhaltern 1, 1' oder Implantatdurchmessern verwendet werden, indem in der Ergebnismatrix 61A, 61 B bzw. in den Zeilen 59A, 60A die entsprechende Anzahl der Markierungen für die Abstandhalter 1, 1'oder Implantatdurchmesser hinterlegt wird.

Für einen effektiven Einsatz in einer Zahnarztpraxis ist es also vorteilhaft, wenn der Anwender mehrere unterschiedliche Abstandhalter 1, 1' zur Verfügung hat, bevorzugt vier bis zehn Abstandhalter 1' zum Bestimmen des Abstands D2 zwischen einer ersten, bereits gebohrten Implantatkavität 54A und einer zweiten, benachbarten, zu bohrenden Implantatkavität 54B und zwei bis vier Abstandhalter 1 zum Bestimmen des Abstands D1 und der Position einer Implantatkavität 54A, die einem lebenden Zahn 55 oder einem bereits am Kiefer befestigten, fertigen Zahnersatz benachbart ist. Die nachfolgende Tabelle gibt für ein bevorzugtes Ausführungsbeispiel mit acht Abstandhaltern 1' (1-7) und drei Abstandhaltern 1 (A - C) die jeweiligen Abstände D2 bzw. D1 an:

| Abstandhalter 1 | A | B | C | | | | | |
|---|---|---|---|---|---|---|---|---|
| Abstand D1 [mm] | 3,8 | 4,3 | 5,6 | | | | | |
| Abstandhalter 1' | 1 | 2 | 3 | 4 | 5 | 6 | 7 | 8 |
| Abstand D2 [mm] | 6,1 | 6,6 | 7,1 | 7,6 | 8,0 | 8,6 | 9,1 | 9,6 |

Bevorzugt sind die Abstandhalter 1, 1' in einem Werkzeugset für Werkzeuge zum Bohren von Implantatkavitäten aufgenommen. Ein bevorzugtes Ausführungsbeispiel eines derartigen Werkzeugsets ist in Figur 3 dargestellt und mit dem Bezugszeichen 62 versehen. Es umfasst mehrere Behältnisse 63, die als Vertiefungen 64 einer Trägerplatte 65 ausgebildet sind. Jede Vertiefung 64 ist so bemessen, dass sie zumindest einen Abstandhalter 1 oder 1' aufnehmen kann, bevorzugt hat jede Vertiefung 64 die Form und Abmessungen eines bestimmten Abstandhalters 1, 1' und bildet damit erste Behältnisse 63A für Abstandhalter 1 und zweite Behältnisse 63B für Abstandhalter 1'. Besonders bevorzugt ist im Boden jedes Behältnisses 63, das einen Abstandhalter 1, 1' aufnimmt, eine erste Bohrung 66 vorgesehen, die, wenn der Abstandhalter 1, 1' im Behältnis 63 aufgenommen ist, unter der Stützvorrichtung 5, 5' und dem Durchstich 7, 7' liegt. Damit kann der Anwender die Abstandhalter 1, 1' ohne diese in die Hand zu nehmen mit dem Dentalinstrument 51 selbst dann verbinden, wenn ein Bohrwerkzeug 50 bereits in dem Dentalinstrument 51 eingespannt ist. Der Anwender schließt dabei Führungseinrichtung 25 an das Dentalinstrument 51 an und drückt dann die Verbindungsvorrichtung 26 der Führungseinrichtung 25 gegen die Verbindungsvorrichtung 9, 9' des im Werkzeugset 62 angeordneten Abstandhalters 1, 1', wobei das Bohrwerkzeug 50 beim Zusammendrücken und der dadurch bewirkten Verschiebung des Instrumentenkopfs 52 mit dem Bohrwerkzeug 50 durch die Kompression der Federelemente 39 durch die Bohrung 66 hindurch tritt.

In den Behältnissen 63B für Abstandhalter 1' ist jeweils eine zweite Bohrung 67 zur Aufnahme des Stifts 13 vorgesehen, wodurch die Positionierung der Abstandhalter 1' in den Behältnissen 63B und der Anschluss an die Verbindungsvorrichtung 26 der Führungseinrichtung 25 verbessert wird.

Werkzeugset 62 umfasst besonders bevorzugt zwei weitere Behältnisse 63, wobei Behältnis 63C zumindest eine implantologische, insbesondere kieferchirurgische Führungseinrichtung, bevorzugt Führungseinrichtung 25, und Behältnis 63D zumindest ein Bohrwerkzeug 50 aufnimmt. Zusätzlich kann zumindest ein weiteres Behältnis zur Aufbewahrung zumindest eines Implantats 70 - 72 vorhanden sein sowie dem Werkzeugset 62 Hilfsmittel 57 zur Auswahl eines Abstandhalters 1, 1' beiliegen. In vorteilhafter und kompakter Weise enthält Werkzeugset 62 damit alle für das Setzen eines Implantats benötigten Werkzeuge.

In einem weiteren bevorzugten Ausführungsbeispiel ist Werkzeugset 62 in einem Behälter, insbesondere einem Metallkästchen mit einem Deckel, aufgenommen. Zwei oder mehr Stützen 68 lagern das Werkzeugset 62 in dem Behälter. Hilfsmittel 57 zur Auswahl eines Abstandhalters 1, 1' kann in diesem Ausführungsbeispiel am Deckel des Behälters angeordnet sein, zum Beispiel durch Aufkleben oder Auflasern.

Die Erfindung ist nicht auf die beschriebenen Ausführungsbeispiele beschränkt, sondern umfaßt alle Ausführungsmöglichkeiten, die das prinzipielle, sinngemäße Funktionsprinzip der Erfindung nicht verändern.

## Patentansprüche

1. Abstandhalter (1, 1') zur Bestimmung der Position einer Implantatkavität (54A, 54B), in die ein Dentalimplantat (70 - 72) einsetzbar ist, wobei der Abstandhalter (1, 1') eine Stützvorrichtung (5, 5') für das Bohrwerkzeug (50), welches die Implantatkavität (54A, 54B) in das Kiefer (56) bohrt, und eine Positioniervorrichtung (2, 2') zur Positionierung des Abstandhalters (1, 1') auf dem Kieferabschnitt (56), an dem die Implantatkavität (54A, 54B) zu setzen ist, umfasst, und der Abstand D1, D2 zwischen der Stützvorrichtung (5,5') und der Positioniervorrichtung (2, 2') feststehend ist,
**dadurch gekennzeichnet,**
- **dass** der Abstand D1 = L_{(1,2,3)}/2 + bB ist, wobei L_{(1,2,3)} gleich dem Durchmessers jenes Implantats (70 - 72) ist, das in die Implantatkavität (54A), die unter Zuhilfenahme des Abstandhalters (1) herstellbar ist, einsetzbar ist, und bB gleich der biologischen Breite von 1,4 - 3,2 mm, bevorzugt 1,5 - 2,1 mm, besonders bevorzugt 1,8 - 2,0 mm ist, oder
- **dass** der Abstand D2 = L_{(1,2,3)}/2 + L_{(1,2,3)}/2 + bB ist, wobei L_{(1,2,3)} gleich dem Durchmesser eines ersten und zweiten Implantats (70 - 72) ist, die in die erste und zweite, nebeneinander angeordnete Implantatkavität (54A, 54B) einsetzbar sind, wobei zumindest eine der beiden lmplantatkavitäten (54A, 54B) unter Verwendung des Abstandhalters (1') herstellbar ist, und bB gleich der biologischen Breite von 1,4 - 3,2 mm, bevorzugt 2,0 - 3,2 mm, besonders bevorzugt 2,5 - 3,0 mm ist.

2. Abstandhalter (1, 1') nach Anspruch 1, **dadurch gekennzeichnet, dass**
mit einem Abstandhalter (1, 1') die Position einer Implantatkavität (54A, 54B) für mehrere Implantate (70 - 72) mit unterschiedlichen Durchmessern (L₁, L₂, L₃) bestimmbar ist, wobei die biologische Breite bB im Bereich von 1,4 - 3,2 mm variiert.

3. Abstandhalter (1) nach Anspruch 1 oder 2, **dadurch gekennzeichnet, dass**
die Positioniervorrichtung (2) als Schulter (3) ausgebildet ist und einen Kontaktpunkt (P) umfasst, mit dem die Schulter (3), sobald der Abstandhalter (1) auf den Kieferabschnitt (56), in dem die Implantatkavität (54A) zu bohren ist, gesetzt ist, einen Zahn (55) oder einen Zahnersatz kontaktiert, der an diesen Kieferabschnitt (56) anschließt, wobei der Abstand D1 der Entfernung zwischen der Mittelachse (11) der Stützvorrichtung (5) und dem Kontaktpunkt (P) entspricht.

4. Abstandhalter (1) nach Anspruch 3, **dadurch gekennzeichnet, dass**
die Schulter (3) kreisförmig oder kreisbogenförmig ausgebildet ist und der Kontaktpunkt (P) am Außenumfang des Kreises oder Kreisbogens angeordnet ist.

5. Abstandhalter (1') nach Anspruch 1 oder 2, **dadurch gekennzeichnet, dass**
die Positioniervorrichtung (2') als Stift (13) ausgebildet ist und der Abstand D2 der Entfernung der Mittelachse (14) des Stifts (13) und der Mittelachse (11') der Stützvorrichtung (5') entspricht.

6. Abstandhalter (1, 1') nach einem der vorhergehenden Ansprüche, **dadurch gekennzeichnet, dass**
die Stützvorrichtung (5, 5') als Durchstich (7, 7') ausgebildet ist, an den eine Anschlusshülse (8, 8') anschließt.

7. Abstandhalter (1, 1') nach Anspruch 6, **dadurch gekennzeichnet, dass**
die Anschlusshülse (8, 8') eine Verbindungsvorrichtung (9, 9') zum Verbinden des Abstandhalters (1, 1') mit einer implantologischen Führungseinrichtung umfasst.

8. Abstandhalter (1, 1') nach Anspruch 7, **gekennzeichnet durch**
eine lösbare Verbindungsvorrichtung (9, 9'), bevorzugt eine Schraub-, Steck-, Rast-oder Klemmverbindung.

9. Abstandhalter (1, 1') nach Anspruch 8, **dadurch gekennzeichnet, dass**
die Anschlusshülse (8, 8') eine Schulter (10, 10') als Teil einer Steck- oder Rastverbindung umfasst.

10. Abstandhalter (1, 1') nach einem der vorhergehenden Ansprüche, **dadurch gekennzeichnet, dass**
der Durchstich (7, 7') mit der Positioniervorrichtung (2, 2') über einen Steg (4, 4') verbunden ist.

11. Abstandhalter (1, 1') nach Anspruch 10, **dadurch gekennzeichnet, dass**
der Steg (4') einen sich zur Stützvorrichtung (5') verjüngenden Abschnitt (4'B) aufweist.

12. Abstandhalter (1, 1') nach Anspruch 10 oder 11, **dadurch gekennzeichnet, dass**
der Steg (4, 4') zumindest eine Aussparung (6, 6') in den Durchstich (7, 7') umfasst.

13. Implantologische, insbesondere kieferchirurgische Führungseinrichtung (25) zur Führung eines in einem Dentalinstrument (51) aufgenommenen Bohrwerkzeugs (50) zum Bohren von Implantatkavitäten (54A, 54B), in die anschließend ein Implantat (70 - 72) einsetzbar ist, mit einem Abstandhalter (1, 1') nach einem der vorhergehenden Ansprüche 1 - 12.

14. Führungseinrichtung (25) nach Anspruch 13, **dadurch gekennzeichnet, dass**
die Führungseinrichtung (25) eine Verbindungsvorrichtung (26) zum Verbinden des Abstandhalters (1, 1') mit der Führungseinrichtung (25) umfasst.

15. Führungseinrichtung (25) nach Anspruch 14, **gekennzeichnet durch**
eine lösbare Verbindungsvorrichtung, bevorzugt eine Schraub-, Steck-, Rast- oder Klemmverbindung.

16. Führungseinrichtung (25) nach Anspruch 15, **dadurch gekennzeichnet, dass**
die Verbindungsvorrichtung (26) zumindest einen Federarm (27) umfasst, und auf jedem Federarm (27) eine Rastnase (29) und ein Rücksprung (30) vorgesehen sind.

17. Führungseinrichtung (25) nach einem der Ansprüche 13 bis 16, **gekennzeichnet durch**
eine Führungshülse (31), die das Bohrwerkzeug (50) aufnimmt und führt, wobei an der Führungshülse (31) zumindest ein Führungsstift (35) zum Führen des Dentalinstruments (51) angeordnet ist.

18. Führungseinrichtung (25) nach Anspruch 17, **dadurch gekennzeichnet, dass**
an der Führungshülse (31) ein Lagerblock (32) vorgesehen ist, an dem der zumindest eine Führungsstift (35) angeordnet ist.

19. Führungseinrichtung (25) nach Anspruch 17 oder 18, **dadurch gekennzeichnet, dass**
der zumindest eine Führungsstift (35) so angeordnet ist, dass zumindest ein Teil des Dentalinstruments (51), bevorzugt der Instrumentenkopf (52), entlang des zumindest einen Führungsstifts (35) verschiebbar ist.

20. Führungseinrichtung (25) nach Anspruch 17 oder 18, **dadurch gekennzeichnet, dass**
der zumindest eine Führungsstift (35) so angeordnet ist, dass das Dentalinstrument (51) mit dem zumindest einen Führungsstift (35) relativ zur Führungshülse (31) verschiebbar ist.

21. Führungseinrichtung (25) nach einem der Ansprüche 17 bis 20, **gekennzeichnet durch**
eine Schiebebuchse (38) als Auflager zumindest eines Teils des zu führenden Dentalinstruments (51), wobei die Schiebebuchse (38) für zumindest einen Führungsstift (35) eine Bohrung (37) aufweist, in der ein Abschnitt eines Führungsstifts (35) aufnehmbar ist.

22. Führungseinrichtung (25) nach Anspruch 21, **gekennzeichnet durch**
zumindest ein Federelement (39), das zwischen der Führungshülse (31) und der Schiebebuchse (38) angeordnet ist und die Schiebebuchse (38) gegen die Führungshülse (31) vorspannt.

23. Führungseinrichtung (25) nach Anspruch 22, **dadurch gekennzeichnet, dass**
mehrere Federelemente (39) vorgesehen sind, die als Spiralfedern (46) ausgebildet sind, und dass jeweils ein Führungsstift (35) im Inneren einer Spiralfeder (46) angeordnet ist, wobei die Führungsstifte (35) durch Bohrungen (34) im Lagerblock (32) ragen, und der Durchmesser der Bohrungen (34) geringer als der Durchmesser der Spiralfedern (46) ist, so dass die Spiralfedern (46) auf der der Schiebebuchse (38) zugewandten Seite des Lagerblocks (32) aufsitzen, so dass, wenn auf die Schiebebuchse (38) eine Kraft einwirkt, die Schiebebuchse (38) mit den Führungsstiften (35) gegen die Federkraft der Spiralfedern (46) in Richtung des Lagerblocks (32) bewegbar ist, wobei die Führungsstifte (35) durch die Bohrungen (34) im Lagerblock (32) gleiten.

24. Führungseinrichtung (25) nach einem der Ansprüche 21 bis 23, **gekennzeichnet durch**
eine Aufnahmevorrichtung (40) für zumindest einen Teil des Dentalinstruments (51), bevorzugt den Instrumentenkopf (52), die mit der Schiebebuchse (38) verbunden ist.

25. Führungseinrichtung (25) nach Anspruch 24, **dadurch gekennzeichnet, dass**
die Aufnahmevorrichtung (40) gegenüber der Schiebebuchse (38) drehbar angeordnet ist.

26. Führungseinrichtung (25) nach Anspruch 24 oder 25, **dadurch gekennzeichnet, dass**
die Aufnahmevorrichtung (40) so ausgebildet ist, dass sie mit dem Dentalinstrument (51) eine lösbare Verbindung eingeht.

27. Führungseinrichtung (25) nach Anspruch 26, **dadurch gekennzeichnet, dass**
die Aufnahmevorrichtung (40) zumindest zwei federnden Flügeln (42) umfasst, zwischen denen der Instrumentenkopf (52) einsteckbar ist, wobei die lichte Weite W zwischen den Flügeln (42) geringer als der Durchmesser des Instrumentenkopfs (52) ist, so dass bei Einstecken des Instrumentenkopfs (52) in die Aufnahmevorrichtung (40) die Flügel (42) radial nach außen federn und das freie Ende (42A) zumindest eines Flügels (42) eine Rastkante (43) aufweist, die, wenn der Instrumentenkopf (52) in die Aufnahmevorrichtung (40) eingesteckt ist, in einen Rücksprung (53) am Instrumentenkopf (52) eingreift.

28. Werkzeugset (62) zur Aufnahme von Werkzeuge zum Bohren von Implantatkavitäten (54A, 54B), in die anschließend ein Implantat (70 - 72) einsetzbar ist, **dadurch gekennzeichnet, dass**
das Werkzeugset (62) zumindest einen Abstandhalter (1, 1') nach einem der Ansprüche 1-12 enthält.

29. Werkzeugset (62) nach Anspruch 28, **dadurch gekennzeichnet, dass**
es des Weiteren zumindest eine Führungseinrichtung (25) nach einen der Ansprüche 13 - 27 und / oder zumindest ein Bohrwerkzeug (50) zum Bohren von Implantatkavitäten (54A, 54B) und / oder Hilfsmittel (57) zur Auswahl eines Abstandhalters (1, 1') und / oder zumindest ein Implantat (70 - 72) enthält.

30. Verfahren zur Herstellung eines Abstandhalters (1, 1') bei dem der Abstand D1, D2 zwischen der Stützvorrichtung (5, 5') für das Bohrwerkzeug (50) und der Positioniervorrichtung (2, 2') unveränderlich ist, **gekennzeichnet durch** die Schritte:
- Bestimmen des Abstands D1 gemäß dem Zusammenhang D1 = L_{(1,2,3)}/2 + bB oder Bestimmen des Abstands D2 gemäß dem Zusammenhang D2 = L_{(1,2,3)}/2 + L_{(1,2,3)}/2 + bB, wobei L_{(1,2,3)} gleich dem Durchmesser eines ersten bzw. eines ersten und zweiten Implantats entspricht und der Wert der biologischen Breite bB zwischen 1,4 - 3,2 mm liegt,
- Zur Verfügung stellen eines Werkstücks,
- Spanabhebende, bevorzugt fräsende, Bearbeitung des Werkstücks, so dass die Positioniervorrichtung (2, 2') im berechneten Abstand D1 oder D2 von der Stützvorrichtung (5, 5') angeordnet ist.
